# EUROPEAN PATENT APPLICATION

(11) **EP 4 759 336 A1**
(43) Date of publication of application: **17.06.2026**
(21) Application number: 24219144.3
(22) Date of filing: 11.12.2024
(51) Int. Cl.: A61L 15/26, A61L 15/32, B33Y 10/00, B33Y 80/00, A61F 13/00

(54) **A COMPOSITE MATERIAL FOR WOUND TREATMENT**

(71) Applicant: MedSkin Solutions Dr. Suwelack AG, 20354 Hamburg (DE)
(72) Inventor: Weinmar, Wolfgang, 48720 Rosendahl (DE); Ningo, Anye, 58739 Wickede (Ruhr) (DE); Wogram, Marco, 21037 Hamburg (DE)
(74) Representative: Kilger, Ute

(57) **Abstract**

The present invention refers to a composite material, a method of producing said composite material as well as its use for treatment of acute wounds, such as surgical and traumatic wounds, and chronic wounds, such as ulcers and other chronic wounds.

## Description

### Technical field

The present invention refers to a composite material, a method of producing said composite material as well as its use in the treatment of acute wounds and chronic wounds.

### Background

Wound healing is a complex and dynamic process that involves the interaction of various cell types, extracellular matrix components, and signaling molecules. Despite advances in medical science, chronic wounds, such as diabetic ulcers, venous leg ulcers, and pressure sores, continue to pose significant challenges in clinical settings. These wounds often fail to proceed through the normal phases of healing, leading to prolonged patient suffering, increased risk of infection, and significant healthcare costs. Traditional wound care treatments, including dressings and topical agents, often prove insufficient for managing these chronic conditions effectively.

Recent developments in biopolymers have shown promise in enhancing wound healing processes. Composite materials, which combine multiple functional components into a single structure, have been explored for their potential to provide a conducive environment for wound healing. These materials can be designed to offer structural support, promote cell proliferation, and deliver therapeutic agents directly to the wound site. However, many existing composite materials have limitations, such as inadequate biocompatibility, insufficient mechanical strength, or suboptimal release profiles of therapeutic agents, which can hinder their effectiveness in clinical applications.

There is a growing need for advanced composite materials that can address the limitations of current wound care treatments. Such materials should ideally support the various stages of wound healing, from hemostasis and inflammation to proliferation and remodeling. They should also be capable of being produced through cost-effective and scalable methods to ensure their widespread adoption in healthcare settings. Additionally, these materials must be versatile enough to be used in different types of wounds, including stagnating wounds, split skin grafts, and ulcers, thereby providing a comprehensive solution for wound management.

The present invention aims to fulfill these requirements by providing a novel composite material. This composite material is designed to enhance the healing of chronic wounds by combining biocompatible materials with bioactive components that promote tissue regeneration and prevent infection. The invention also includes a method for producing this composite material, ensuring that it can be manufactured efficiently and consistently. The use of this composite material in treating acute wounds (e.g. surgical and traumatic wounds) and chronic wounds (ulcers and other chronic wounds) represents a significant advancement in the field of wound care, offering new hope for patients suffering from these challenging conditions.

### Description of the invention

In one aspect, the present invention refers to a composite material comprising
- a material layer comprising a plurality of cells, wherein each cell is a spatially confined area, and
- a matrix layer,

wherein said material layer comprises an open area in said cells, and wherein said material layer exhibits a proportion of the open area to the entire surface area of the material layer in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm,
wherein said matrix layer comprises a biopolymer selected from the group consisting of collagen, hyaluronic acid, polyacrylate, and polysaccharides, such as alginic acid, nanocellulose, carboxymethyl cellulose or chitosan, or mixtures thereof or salts thereof, and wherein said material layer and said matrix layer are adhered to one another by means of a mechanical adhesive force of from 0.05 N to 2 N.

The material layer, such as a medical grade silicone grid, serves to control moisture loss from the wound providing a flexible adherent covering for the wound surface and adding increased mechanical strength to the device.

The matrix layer serves as a scaffold for cellular repopulation and capillary ingrowth to support the regeneration of neodermis, the reduction of scarring and the prevention of wound contraction. For the reconstruction of mucosal defects, the aim of the treatment is to improve the quality of reconstructed tissue and reduce scarring.

After application to a wound bed the composite material according to the present invention forms a soft, pliable moist gel sheet at the wound surface. The composite material provides an ideal environment for natural wound healing. It serves as a scaffold in the reconstitution process of dermal structures and thus skin reconstruction. It is conducive to modulation of wound contraction, scar tissue formation and thereby increasing elasticity of reconstructed skin.

The porous microstructure of the product allows good re-vascularization and ingrowths of local dermal fibroblasts. Moreover, the composite material has excellent hemostatic properties and thus reduces the risk of hematoma formation beneath the split-skin sub-graft or between the matrix and the wound bed.

An additional advantage of the composite material according to the present invention is that it provides a scaffold that enhances cellular migration and tissue regeneration. This can lead to faster recovery times compared to traditional methods.

In one embodiment, the thickness of the matrix layer in said composite material is in the range of from 0.50 to 5.0 mm, preferably from 0.75 to 3.50 mm.

In one embodiment, said material layer in the composite material has a proportion of the open area to the entire surface area in the range of from 10% to 20%. In one embodiment, said material layer in the composite material has a proportion of the open area to the entire surface area in the range of from 20% to 30%.

In one embodiment, said material layer in the composite material has a proportion of the open area to the entire surface area in the range of from 10% to 50% and a thickness in the range of from 0.1 to 2 mm. In one embodiment, said material layer in the composite material has a proportion of the open area to the entire surface area in the range of from 20% to 40% and a thickness in the range of from 0.30 to 0.70 mm.

In one embodiment said biopolymer is collagen. In one embodiment said biopolymer is alginic acid or a salt thereof, such as calcium alginate, or sodium alginate. In one embodiment said biopolymer is hyaluronic acid or a salt thereof, such as sodium hyaluronate, potassium hyaluronate or calcium hyaluronate. In one embodiment said biopolymer is chitosan or a salt thereof, such as chitosan hydrochloride, chitosan acetate or chitosan lactate. In one embodiment said biopolymer is carboxymethyl cellulose or a salt thereof, such as sodium carboxymethyl cellulose, calcium carboxymethyl cellulose or potassium carboxymethyl cellulose.

In one embodiment, said biopolymer in the matrix layer comprises collagen, alginic acid, or mixtures or salts thereof.

In one embodiment, said biopolymer in the matrix layer comprises collagen by weight in the range of from 80 to 98% and alginic acid or a salt thereof, such as calcium alginate by weight in the range of from 2 to 20%. In one embodiment, said biopolymer in the matrix layer comprises collagen by weight in the range of from 70 to 90% and alginic acid or a salt thereof, such as calcium alginate by weight in the range of from 10 to 30%. In one embodiment, said biopolymer in the matrix layer comprises collagen by weight in the range of from 60 to 80% and alginic acid or a salt thereof, such as calcium alginate by weight in the range of from 20 to 40%. In one embodiment, said biopolymer in the matrix layer comprises collagen by weight in the range of from 50 to 70% and alginic acid or a salt thereof, such as calcium alginate by weight in the range of from 30 to 50%. In one embodiment, said biopolymer in the matrix layer comprises collagen by weight in the range of from 40 to 60% and alginic acid or a salt thereof, such as calcium alginate by weight in the range of from 40 to 60%. In one embodiment, said biopolymer in the matrix layer comprises collagen by weight in the range of from 30 to 50% and alginic acid or a salt thereof, such as calcium alginate by weight in the range of from 50 to 70%. In one embodiment, said biopolymer in the matrix layer comprises collagen by weight in the range of from 20 to 40% and alginic acid or a salt thereof, such as calcium alginate by weight in the range of from 60 to 80%. In one embodiment, said biopolymer in the matrix layer comprises collagen by weight in the range of from 10 to 30% and alginic acid or a salt thereof, such as calcium alginate by weight in the range of from 70 to 90%. In one embodiment, said biopolymer in the matrix layer comprises collagen by weight in the range of from 2 to 20% and alginic acid or a salt thereof, such as calcium alginate by weight in the range of from 80 to 98%.

In one embodiment, said biopolymer in the matrix layer comprises collagen, hyaluronic acid, or mixtures or salts thereof.

In one embodiment, said biopolymer in the matrix layer comprises collagen by weight in the range of from 80 to 98% and hyaluronic acid by weight in the range of from 2 to 20%. In one embodiment, said biopolymer in the matrix layer comprises collagen by weight in the range of from 70 to 90% and hyaluronic acid by weight in the range of from 10 to 30%. In one embodiment, said biopolymer in the matrix layer comprises collagen by weight in the range of from 60 to 80% and hyaluronic acid by weight in the range of from 20 to 40%. In one embodiment, said biopolymer in the matrix layer comprises collagen by weight in the range of from 50 to 70% and hyaluronic acid by weight in the range of from 30 to 50%. In one embodiment, said biopolymer in the matrix layer comprises collagen by weight in the range of from 40 to 60% and hyaluronic acid by weight in the range of from 40 to 60%. In one embodiment, said biopolymer in the matrix layer comprises collagen by weight in the range of from 30 to 50% and hyaluronic acid by weight in the range of from 50 to 70%. In one embodiment, said biopolymer in the matrix layer comprises collagen by weight in the range of from 20 to 40% and hyaluronic acid by weight in the range of from 60 to 80%. In one embodiment, said biopolymer in the matrix layer comprises collagen by weight in the range of from 10 to 30% and hyaluronic acid by weight in the range of from 70 to 90%. In one embodiment, said biopolymer in the matrix layer comprises collagen by weight in the range of from 2 to 20% and hyaluronic acid by weight in the range of from 80 to 98%.

In one embodiment, said biopolymer in the matrix layer comprises collagen, carboxymethyl cellulose, or mixtures or salts thereof.

In one embodiment, said biopolymer in the matrix layer comprises collagen by weight in the range of from 80 to 98% and carboxymethyl cellulose by weight in the range of from 2 to 20%. In one embodiment, said biopolymer in the matrix layer comprises collagen by weight in the range of from 70 to 90% and carboxymethyl cellulose by weight in the range of from 10 to 30%. In one embodiment, said biopolymer in the matrix layer comprises collagen by weight in the range of from 60 to 80% and carboxymethyl cellulose by weight in the range of from 20 to 40%. In one embodiment, said biopolymer in the matrix layer comprises collagen by weight in the range of from 50 to 70% and carboxymethyl cellulose by weight in the range of from 30 to 50%. In one embodiment, said biopolymer in the matrix layer comprises collagen by weight in the range of from 40 to 60% and carboxymethyl cellulose by weight in the range of from 40 to 60%. In one embodiment, said biopolymer in the matrix layer comprises collagen by weight in the range of from 30 to 50% and carboxymethyl cellulose by weight in the range of from 50 to 70%. In one embodiment, said biopolymer in the matrix layer comprises collagen by weight in the range of from 20 to 40% and carboxymethyl cellulose by weight in the range of from 60 to 80%. In one embodiment, said biopolymer in the matrix layer comprises collagen by weight in the range of from 10 to 30% and carboxymethyl cellulose by weight in the range of from 70 to 90%. In one embodiment, said biopolymer in the matrix layer comprises collagen by weight in the range of from 2 to 20% and carboxymethyl cellulose by weight in the range of from 80 to 98%.

In one embodiment, said biopolymer in the matrix layer comprises collagen, chitosan, or mixtures or salts thereof.

In one embodiment, said biopolymer in the matrix layer comprises collagen by weight in the range of from 80 to 98% and chitosan by weight in the range of from 2 to 20%. In one embodiment, said biopolymer in the matrix layer comprises collagen by weight in the range of from 70 to 90% and chitosan by weight in the range of from 10 to 30%. In one embodiment, said biopolymer in the matrix layer comprises collagen by weight in the range of from 60 to 80% and chitosan by weight in the range of from 20 to 40%. In one embodiment, said biopolymer in the matrix layer comprises collagen by weight in the range of from 50 to 70% and chitosan by weight in the range of from 30 to 50%. In one embodiment, said biopolymer in the matrix layer comprises collagen by weight in the range of from 40 to 60% and chitosan by weight in the range of from 40 to 60%. In one embodiment, said biopolymer in the matrix layer comprises collagen by weight in the range of from 30 to 50% and chitosan by weight in the range of from 50 to 70%. In one embodiment, said biopolymer in the matrix layer comprises collagen by weight in the range of from 20 to 40% and chitosan by weight in the range of from 60 to 80%. In one embodiment, said biopolymer in the matrix layer comprises collagen by weight in the range of from 10 to 30% and chitosan by weight in the range of from 70 to 90%. In one embodiment, said biopolymer in the matrix layer comprises collagen by weight in the range of from 2 to 20% and chitosan by weight in the range of from 80 to 98%.

In one embodiment, the matrix layer in said composite material comprises or essentially consists of collagen. In one embodiment, the matrix layer in said composite material comprises at least 90% by weight collagen, preferably the matrix layer in said composite material comprises at least 95% by weight collagen. In one embodiment, the matrix layer in said composite material comprises collagen by weight in the range of from 85% to 97%, preferably from 90% to 97%.

In one embodiment, the matrix layer in said composite material comprises or essentially consists of alginic acid or a salt thereof, such as calcium alginate. In one embodiment, the matrix layer in said composite material comprises at least 90% by weight alginic acid or a salt thereof, such as calcium alginate, preferably the matrix layer in said composite material comprises at least 95% by weight alginic acid or a salt thereof, such as calcium alginate. In one embodiment, the matrix layer in said composite material comprises alginic acid or a salt thereof, such as calcium alginate, by weight in the range of from 85% to 97%, preferably from 90% to 97%.

In one embodiment, the matrix layer in said composite material comprises or essentially consists of hyaluronic acid. In one embodiment, the matrix layer in said composite material comprises at least 90% by weight hyaluronic acid, preferably the matrix layer in said composite material comprises at least 95% by weight hyaluronic acid. In one embodiment, the matrix layer in said composite material comprises hyaluronic acid by weight in the range of from 85% to 97%, preferably from 90% to 97%.

In one embodiment, the matrix layer in said composite material comprises or essentially consists of carboxymethyl cellulose. In one embodiment, the matrix layer in said composite material comprises at least 90% by weight carboxymethyl cellulose, preferably the matrix layer in said composite material comprises at least 95% by weight carboxymethyl cellulose. In one embodiment, the matrix layer in said composite material comprises carboxymethyl cellulose by weight in the range of from 85% to 97%, preferably from 90% to 97%.

In one embodiment, the matrix layer in said composite material comprises or essentially consists of chitosan. In one embodiment, the matrix layer in said composite material comprises at least 90% by weight chitosan, preferably the matrix layer in said composite material comprises at least 95% by weight chitosan. In one embodiment, the matrix layer in said composite material comprises chitosan by weight in the range of from 85% to 97%, preferably from 90% to 97%.

In one embodiment, the matrix layer further comprises elastin. In one embodiment, the matrix layer in said composite material comprises elastin in an amount of from 1% to 10%, preferably from 1% to 5%, more preferably 1.5% to 3.0%, in particular, from 1.80 to 2.80 % by weight based on the total weight of the matrix layer.

In one embodiment, the matrix layer in said composite material comprises collagen by weight in the range of from 90% to 99% and elastin by weight from 1% to 10%. In one embodiment, the matrix layer in said composite material comprises collagen by weight in the range of from 95% to 99% and elastin by weight from 1% to 5%. In one embodiment, the matrix layer in said composite material comprises collagen by weight in the range of from 97.0% to 98.5% and elastin by weight from 1.5% to 3.0%. In one embodiment, the matrix layer in said composite material comprises collagen by weight in the range of from 97.2% to 98.2% and elastin by weight from 1.8% to 2.8%.

In one embodiment, the collagen is selected from the group consisting of bovine collagen, fish collagen, porcine collagen, sheep collagen, human collagen, vegetarian collagen or fungal collagen.

In one embodiment, said collagen is derived from bovine skin. Bovine skin is a widely recognized and abundant source of high-quality collagen, known for its effectiveness in promoting wound healing due to its structural similarity to human collagen.

In one embodiment, said collagen is sourced from fish scales. Collagen from fish scales offers a unique composition with a smaller molecular size compared to mammalian collagen, which can enhance its absorption and integration into human tissues.

In one embodiment, said collagen is extracted from eggshell membranes.

In one embodiment, said collagen is obtained from mollusk shells.

In one embodiment, said collagen is derived from other animal sources, such as sheep skin, porcine skin or chicken cartilage.

In one embodiment, said collagen is derived from non-animal origins, such as mushrooms (fungal collagen).

In one embodiment the collagen is selected from the group comprising collagen type I, collagen type III, collagen type V or a mixtures thereof.

In one embodiment, said biopolymer in the matrix layer comprises collagen type I and collagen type III.

In one embodiment, said biopolymer in the matrix layer comprises collagen type I by weight in the range of from 80 to 98% and collagen type III by weight in the range of from 2 to 20%. In one embodiment, said biopolymer in the matrix layer comprises collagen type I by weight in the range of from 70 to 90% and collagen type III by weight in the range of from 10 to 30%. In one embodiment, said biopolymer in the matrix layer comprises collagen type I by weight in the range of from 60 to 80% and collagen type III by weight in the range of from 20 to 40%. In one embodiment, said biopolymer in the matrix layer comprises collagen type I by weight in the range of from 50 to 70% and collagen type III by weight in the range of from 30 to 50%. In one embodiment, said biopolymer in the matrix layer comprises collagen type I by weight in the range of from 40 to 60% and collagen type III by weight in the range of from 40 to 60%. In one embodiment, said biopolymer in the matrix layer comprises collagen type I by weight in the range of from 30 to 50% and collagen type III by weight in the range of from 50 to 70%. In one embodiment, said biopolymer in the matrix layer comprises collagen type I by weight in the range of from 20 to 40% and collagen type III by weight in the range of from 60 to 80%. In one embodiment, said biopolymer in the matrix layer comprises collagen type I by weight in the range of from 10 to 30% and collagen type III by weight in the range of from 70 to 90%. In one embodiment, said biopolymer in the matrix layer comprises collagen type I by weight in the range of from 2 to 20% and collagen type III by weight in the range of from 80 to 98%.

In one embodiment, said biopolymer in the matrix layer comprises collagen type I and collagen type V.

In one embodiment, said biopolymer in the matrix layer comprises collagen type I by weight in the range of from 80 to 98% and collagen type V by weight in the range of from 2 to 20%. In one embodiment, said biopolymer in the matrix layer comprises collagen type I by weight in the range of from 70 to 90% and collagen type V by weight in the range of from 10 to 30%. In one embodiment, said biopolymer in the matrix layer comprises collagen type I by weight in the range of from 60 to 80% and collagen type V by weight in the range of from 20 to 40%. In one embodiment, said biopolymer in the matrix layer comprises collagen type I by weight in the range of from 50 to 70% and collagen type V by weight in the range of from 30 to 50%. In one embodiment, said biopolymer in the matrix layer comprises collagen type I by weight in the range of from 40 to 60% and collagen type V by weight in the range of from 40 to 60%. In one embodiment, said biopolymer in the matrix layer comprises collagen type I by weight in the range of from 30 to 50% and collagen type V by weight in the range of from 50 to 70%. In one embodiment, said biopolymer in the matrix layer comprises collagen type I by weight in the range of from 20 to 40% and collagen type V by weight in the range of from 60 to 80%. In one embodiment, said biopolymer in the matrix layer comprises collagen type I by weight in the range of from 10 to 30% and collagen type V by weight in the range of from 70 to 90%. In one embodiment, said biopolymer in the matrix layer comprises collagen type I by weight in the range of from 2 to 20% and collagen type V by weight in the range of from 80 to 98%.

In one embodiment, said biopolymer in the matrix layer comprises collagen types III and collagen type V.

In one embodiment, said biopolymer in the matrix layer comprises collagen type III by weight in the range of from 80 to 98% and collagen type V by weight in the range of from 2 to 20%. In one embodiment, said biopolymer in the matrix layer comprises collagen type III by weight in the range of from 70 to 90% and collagen type V by weight in the range of from 10 to 30%. In one embodiment, said biopolymer in the matrix layer comprises collagen type III by weight in the range of from 60 to 80% and collagen type V by weight in the range of from 20 to 40%. In one embodiment, said biopolymer in the matrix layer comprises collagen type III by weight in the range of from 50 to 70% and collagen type V by weight in the range of from 30 to 50%. In one embodiment, said biopolymer in the matrix layer comprises collagen type III by weight in the range of from 40 to 60% and collagen type V by weight in the range of from 40 to 60%. In one embodiment, said biopolymer in the matrix layer comprises collagen type III by weight in the range of from 30 to 50% and collagen type V by weight in the range of from 50 to 70%. In one embodiment, said biopolymer in the matrix layer comprises collagen type III by weight in the range of from 20 to 40% and collagen type V by weight in the range of from 60 to 80%. In one embodiment, said biopolymer in the matrix layer comprises collagen type III by weight in the range of from 10 to 30% and collagen type V by weight in the range of from 70 to 90%. In one embodiment, said biopolymer in the matrix layer comprises collagen type III by weight in the range of from 2 to 20% and collagen type V by weight in the range of from 80 to 98%.

In one embodiment, the matrix layer in said composite material has a thickness in the range of from 1 mm to 3 mm.

In one embodiment, the matrix layer in said composite material has a density of from 0.018 to 0.034 g/cm³, preferably from 0.020 to 0.030 g/cm³.

In one embodiment, the matrix layer in said composite material has a pH of from 2.5 to 7.0, preferably from 2.5 to 5.5, most preferred from 2.8 to 3.4.

In one embodiment, said material layer and said matrix layer in said composite material are adhered to one another by means of a mechanical adhesive force of from 0,05 N to 2 N. Said mechanical force can be measured by the method as described in Example 2.

In one embodiment, said matrix layer is detachable from said material layer when force 0,2N pro 1 mm of thickness is applied (tear strength test). Said tear strength can be measured by the method as provided in Example 3.

In one embodiment, the material layer and the matrix layer in said composite material are arranged such that the matrix layer is between a wound and the material layer. So, the material layer and the matrix layer in said composite material are arranged such that the material layer is not in physical contact with skin or wound. In said composite material the material layer and the matrix layer are arranged such that the material layer is spaced apart from skin or the wound.

In one embodiment, the material layer and the matrix layer in said composite material are not bound to one another with a cross-linking agent.

In one embodiment, the material layer and the matrix layer in said composite material are not bound to one another by covalent bonds.

In one embodiment, said material layer is characterized by the distribution of cells of from 25 to 70 cells, preferably from 40 to 60 cells, more preferably from 45 to 55 cells per 1 cm² of the surface area of the material layer.

In one embodiment, said material layer is characterized by the elastic modulus of from 60 kPa to 320 kPa, preferably from 100 kPa to 300 kPa.

In one embodiment, the plurality of cells in said material layer are arranged in a grid, preferably a silicone grid. In a preferred embodiment the plurality of cells in said material layer are arranged in a regularly repeating grid pattern, preferably regularly repeating silicone grid pattern.

In one embodiment, the plurality of cells in said material layer comprise square-shaped cells.

In one embodiment, the average distance between two non-crossing walls of the grid in said material layer is in the range of from 0.90 to 1.30 mm, preferably of from 1.00 to 1.20 mm.

In one embodiment, the material layer in said composite material comprises or essentially consists of silicone or polyurethane. In one embodiment, the material layer in said composite material comprises or essentially consists of silicone. In one embodiment, the material layer in said composite material comprises or essentially consists of polyurethane.

In one embodiment, the material layer comprises at least 90% by weight silicone, preferably the material layer comprises at least 95% by weight silicone, more preferably the material layer comprises at least 96% by weight silicone, more preferably the material layer comprises at least 97% by weight silicone, more preferably the material layer comprises at least 98% by weight silicone, most preferred the material layer comprises at least 99% by weight silicone.

In one embodiment, the material layer comprises silicone by weight in the range of from 90 to 99.5%, preferably in the range of from 95 to 99%.

In one embodiment, the material layer comprises at least 90% by weight polyurethane, preferably the material layer comprises at least 95% by weight polyurethane, more preferably the material layer comprises at least 96% by weight polyurethane, more preferably the material layer comprises at least 97% by weight polyurethane, more preferably the material layer comprises at least 98% by weight polyurethane, most preferred the material layer comprises at least 99% by weight polyurethane.

In one embodiment, the material layer comprises polyurethane by weight in the range of from 90 to 99.5%, preferably in the range of from 95 to 99%.

In one embodiment, the silicone in said material layer is medical grade certified for biocompatibility (ISO 10993) USP Class VI.

USP Class VI relates to in vivo biological reactivity tests, its purpose is to determine the biological response impact of elastomeric materials on live animals. To achieve USP Class VI certification, the material goes through a series of biological tests: (1) Systemic toxicity, (2) Intracutaneous reactivity, and (3) Muscle implantation.

### Systemic Injection Test

Sample of the compound is prepared with specific extraction fluids like vegetable oil and polyethylene glycol is applied to the skin, inhaled, or administered orally. This test measures toxicity and irritation. The specification is met when extracts of the test item do not produce significantly greater system reaction than the blank extractant.

### Intracutaneous Test

Sample of the compound is in contact with live subdermal tissue (the tissue which the medical device/equipment is planned to contact). This test measures toxicity and localized irritation. The specification is met when extracts of the test item do not produce significantly greater tissue reaction than the blank extractant.

### Implantation

Intramuscular implantation of the compound into the specimen. This test measures toxicity, infection, and irritation. The specification is met when the macroscopic reaction of the test item is not significant compared to the USP negative control plastic after one week implantation.

In one embodiment, the material layer in said composite material is essentially flat.

In another aspect, the invention refers to the composite material according to any of the above embodiments for use as a medicament. In one embodiment the invention refers to the composite material according to any of the above embodiments for use in the treatment of acute wounds, such as surgical and traumatic wounds, and chronic wounds, such as ulcers and other chronic wounds.

In another aspect, the invention refers to the use of the composite material according to any of the above embodiments in the treatment of acute wounds, such as surgical and traumatic wounds, and chronic wounds, such as ulcers and other chronic wounds.

In another aspect, the invention refers to the method of treatment of acute wounds, such as surgical and traumatic wounds, and chronic wounds, such as ulcers and other chronic wounds, comprising administering the composite material according to any of the above embodiments to the subject in need thereof.

In another aspect, the invention refers to a wound dressing, comprising the composite material according to any of the above embodiments.

In another aspect, the invention refers to a method for producing a composite material as defined in any of the above embodiments, comprising the following steps:
a) Providing a suspension of a biopolymer in an aqueous solution, wherein the concentration of the biopolymer in said suspension is in the range of from 1.0 to 4.0%, preferably from 1.40% to 2.00% more preferably from 1.55 to 1.80% with respect to the total weight of said suspension, wherein the biopolymer is selected from the group consisting of collagen, hyaluronic acid, polyacrylate, and polysaccharides, such as alginic acid, nanocellulose, carboxymethyl cellulose or chitosan, or mixtures thereof or salts thereof;
b) Shaping said suspension to provide a matrix layer;
c) Providing a material which is characterized by the elastic modulus of from 60 kPa to 320 kPa, preferably from 100 kPa to 300 kPa;
d) Applying said material onto said matrix layer using a computer numerical control (CNC) machine to provide a composite material, wherein said CNC machine is configured to provide a material layer in said composite material with a proportion of open area to the entire surface area in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm; and
e) Vulcanizing the material obtained in step d), in particular by exposing said material to an elevated temperature and/or UV irradiation.

In a preferred embodiment of said method, the biopolymer in step a) comprises or essentially consists of collagen. In one embodiment, the biopolymer in step a) comprises at least 90% by weight collagen, preferably at least 95% by weight collagen, more preferably at least 97% by weight of collagen, even more preferably at least 98% by weight of collagen, most preferred at least 99% by weight of collagen.

In one embodiment of said method, said aqueous solution in step a) is water.

In one embodiment of said method, the pH of said suspension obtained in step a) is further adjusted to the range of from 3.0 to 3.4.

In one embodiment of said method, the suspension provided in step a) is mixed with elastin. In one embodiment, the suspension provided in step a) is mixed with elastin in a vacuum mixer.

In one embodiment, the suspension provided in step a) is mixed with elastin in the proportion of 90% to 99% of the biopolymer, such as collagen, to 1% to 10% of elastin by weight. In one embodiment, the suspension provided in step a) is mixed with elastin in the proportion of 95% to 99% of the biopolymer, such as collagen, to 1% to 5% of elastin by weight. In one embodiment, the suspension provided in step a) is mixed with elastin in the proportion of 97.0% to 98.5% of the biopolymer, such as collagen, to 1.5% to 3.0% of elastin by weight. In one embodiment, the suspension provided in step a) is mixed with elastin in the proportion of 97.2% to 98.2% of the biopolymer, such as collagen, to 1.8% to 2.8% of elastin by weight.

In one embodiment of said method, said suspension in step b) is shaped in a form of sheets.

In one embodiment of said method, said shaping in step b) comprises casting the suspension obtained in step a), optionally mixed with elastin, into molds followed by freeze-drying.

In one embodiment of said method, the material in step c) is silicone or polyurethane. In one embodiment of said method, the material in step c) is silicone. In one embodiment of said method, the material in step c) is polyurethane.

In one embodiment, vulcanization in step e) is conducted by heating the material at a temperature in the range of from 60°C to 110°C for a period in the range of from 0.25 to 2 hours.

In one embodiment, said silicone or polyurethane is certified for biocompatibility (ISO 10993) USP Class VI.

In one embodiment of said method, the composite material obtained in step e) is further cut into sheets, optionally by a slicing machine.

In another aspect, the invention refers to a composite material which is obtainable by the method according to any of the above embodiments.

With the above context, the following consecutively numbered embodiments provide further specific aspects of the invention:
1. A composite material comprising
   - a material layer comprising a plurality of cells, wherein each cell is a spatially confined area, and
   - a matrix layer,
      wherein said material layer comprises an open area in said cells, and wherein said material layer exhibits a proportion of the open area to the entire surface area of the material layer in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm,
      wherein said matrix layer comprises a biopolymer selected from the group consisting of collagen, hyaluronic acid, polyacrylate, and polysaccharides, such as alginic acid, nanocellulose, carboxymethyl cellulose or chitosan, or mixtures thereof or salts thereof, and
      wherein said material layer and said matrix layer are adhered to one another by means of a mechanical adhesive force of from 0.05 N to 2 N.
2. The composite material according to embodiment 1, wherein said material layer and said matrix layer are arranged such that the material layer is spaced apart from skin or a wound when applied to the skin.
3. The composite material according to embodiment 1 or 2, wherein said material layer and said matrix layer are not bound to one another with a cross-linking agent.
4. The composite material according to any of embodiments 1 to 3, wherein said material layer is characterized by the distribution of cells of from 25 to 70 cells, preferably from 40 to 60 cells, more preferably from 45 to 55 cells per 1 cm² of the surface area of the material layer.
5. The composite material according to any of embodiments 1 to 4, wherein said material layer is characterized by the elastic modulus of from 60 to 320 kPa, preferably of from 100 kPa to 300 kPa.
6. The composite material according to any of embodiments 1 to 5, wherein the plurality of cells in said material layer are arranged in a grid.
7. The composite material according to any of embodiments 1 to 6, wherein the plurality of cells in said material layer comprise square-shaped cells.
8. The composite material according to embodiments 6 or 7, wherein the average distance between two non-crossing walls of the grid in said material layer is in the range of from 0.90 to 1.30 mm, preferably of from 1.00 to 1.20 mm.
9. The composite material according to any of embodiments 1 to 8, wherein said material layer comprises or essentially consists of silicone or polyurethane.
10. The composite material according to embodiment 9, wherein said material layer comprises or essentially consists of silicone.
11. The composite material according to any of embodiments 1 to 10, wherein said matrix layer comprises or essentially consists of collagen.
12. The composite material according to embodiments 11, wherein said matrix layer comprises collagen in an amount of from 85% to 97%, preferably from 90% to 97%, by weight based on the total weight of the matrix layer.
13. The composite material according to any of embodiments 1 to 12, wherein said matrix layer further comprises elastin.
14. The composite material according to embodiment 13, wherein said matrix layer comprises elastin in an amount of from 1% to 10%, preferably from 1% to 5%, more preferably 1.5% to 3.0%, in particular from 1.80 to 2.80 % by weight based on the total weight of the matrix layer.
15. The composite material according to any of embodiments 1 to 14, wherein said matrix layer has a thickness in the range of from 1 mm to 3 mm.
16. The composite material according to any of embodiments 1 to 15, wherein said matrix layer has a density of from 0.018 to 0.034 g/cm³, preferably from 0.020 to 0.030 g/cm³.
17. The composite material according to any of embodiments 1 to 16, wherein said matrix layer has a pH in the range of from 2.5 to 7.0, preferably in the range of from 2.5 to 5.5, most preferred in the range of from 2.8 to 3.4.
18. The composite material according to any of embodiments 1 to 17, wherein the collagen is selected from the group comprising collagen Type I, collagen Type III, collagen Type V or a mixture thereof
19. The composite material according to any of embodiments 9 to 18, wherein the silicone or polyurethane is medical grade certified for biocompatibility (ISO 10993) USP Class VI.
20. The composite material according to any of embodiments 1 to 19, wherein said material layer is essentially flat.
21. The composite material according to any of embodiments 1 to 20 for use as a medicament.
22. The composite material according to any of embodiments 1 to 21 for use in the treatment of acute wounds, such as surgical and traumatic wounds, and chronic wounds, such as ulcers and other chronic wounds.
23. A method for producing a composite material as defined in any of the above embodiments, comprising the following steps:
   a) Providing a suspension of a biopolymer in an aqueous solution, wherein the concentration of the biopolymer in said suspension is in the range of from 1.0 to 4.0%, preferably from 1.40% to 2.00% more preferably from 1.55 to 1.80% with respect to the total weight of said suspension, wherein the biopolymer is selected from the group consisting of collagen, hyaluronic acid, polyacrylate, and polysaccharides, such as alginic acid, nanocellulose, carboxymethyl cellulose or chitosan, or mixtures thereof or salts thereof;
   b) Shaping said suspension to provide a matrix layer;
   c) Providing a material which is characterized by the elastic modulus of from 60 kPa to 320 kPa, preferably from 100 kPa to 300 kPa;
   d) Applying said material onto said matrix layer using a computer numerical control (CNC) machine to provide a composite material, wherein said CNC machine is configured to provide a material layer in said composite material with a proportion of open area to the entire surface area in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm; and
   e) Vulcanizing the material obtained in step d), in particular by exposing said material to an elevated temperature and/or UV Irradiation.

### Definitions

Listed below are definitions of various terms used to describe this invention. These definitions apply to the terms as they are used throughout this specification and claims unless otherwise limited in specific instances either individually or as part of a larger group. Unless defined otherwise all technical and scientific terms used herein generally have the same meaning as commonly understood by one of ordinary skill in the art to which this invention belongs.

As used herein, the articles "a" and "an" refer to one or to more than one (i.e. to at least one) of the grammatical object of the article. By way of example, "an element" means at least one element, i.e. an element or more than one element.

As used herein, the term "spacially confined area" refers to an area that is enclosed by boundaries, referred to as walls. These walls delineate the extent of the area and separate it from adjacent areas. Each wall has a defined width, which ranges from 0.1 to 10 mm, preferably from 0.1 to 5 mm, more preferably from 0.1 to 3 mm, even more preferably from 0.2 to 1 mm, most preferred from 0.3 to 1.0 mm. This range ensures that the walls are physically significant and distinguishable. The term "open area" refers to the portion of the spatially confined area that is within the boundaries of the walls but does not include the walls themselves. The term "entire surface area" includes both the open area and the area occupied by the walls. It is the total spatial extent of the confined area, combining the walls and the space they enclose. Two or more cells, each being a spatially confined area, may share a common wall. This shared wall may be wholly or partially common to the adjoining cells, facilitating the formation of a gridlike structure with interconnected cells.

Unless specifically defined, the term "thickness" related to a material layer or a matrix layer refers to an average thickness of said layer.

As used herein, the term "essentially consists" refers to a composition or a material that contains at least 90%, preferably for at least 95%, and most preferred at least 98% of a specified component by weight. This term indicates that the specified component predominates in the composition, with minimal presence of other substances.

As used herein, the term "essentially flat" refers to the material with the thickness throughout the length and the width not deviating from the average thickness by more than ±20%, preferably ± 10%. For example, with an average thickness of 1 mm the thickness of the material throughout its length and width remains in the range from 0.8 to 1.2 mm, preferably in the range of from 0.9 to 1.1 mm. With an average thickness of 2 mm the thickness of the essentially flat material throughout its length and width remains in the range of from 1.6 to 2.4 mm, preferably in the range of from 1.8 to 2.2 mm.

As used herein, the term "sheet" refers to an essentially flat material, wherein the thickness of the material is in the range of from 1 to 8 mm.

As used herein, the term "salt" refers to ionic a chemical compound consisting of an ionic assembly of a positively charged cation and a negatively charged anion.

As used herein, the term "polysaccharide" refers to polymers comprising a backbone comprised mainly of (at least 90%) monosaccharide repeating units and/or derivatized monosaccharide repeating units.

As used herein, the term "alginic acid" refers to an anionic polysaccharide composed of β-D-mannuronic acid and α-L-guluronic acid units bound by means of 1-4 glycosidic bonds. It forms a viscous solution when hydrated and can cross-link with divalent cations to form gels. As used herein, the term "alginate" refers to the anion of alginic acid. Therefore, the terms "alginate" and "alginate salt" are used interchangeable in the context of the present invention. The alginate salt can be, for example, calcium alginate.

As used herein, the term "collagen" refers to a natural fibrous protein composed primarily of glycine, proline, and hydroxyproline, forming triple helical structures. Three collagen polypeptide chains ("α-chains") are wound around each other to form this helical molecule.

As used herein, the term "hyaluronic acid" refers to a glycosaminoglycan composed of repeating disaccharide units of glucuronic acid and N-acetylglucosamine. It is a linear polysaccharide known for its high molecular weight and hydrophilic properties.

As used herein, the term "polyacrylate" refers to a polymer made from the polymerization of acrylic acid or its esters, characterized by repeating acrylate units. These polymers can be tailored with various side chains to adjust their physical and chemical properties.

As used herein, the term "nanocellulose" refers to cellulose in the form of nanometer-sized fibrils or crystals, produced by mechanical or chemical processing of cellulose fibers.

As used herein, the term "carboxymethyl cellulose" refers to a cellulose derivative where carboxymethyl groups are substituted on the hydroxyl groups of the glucopyranose monomers.

As used herein, the term "chitosan" refers to a linear polysaccharide composed of randomly distributed β-(1→4)-linked D-glucosamine and N-acetyl-D-glucosamine. It is derived from chitin by deacetylation and is soluble in acidic solutions.

As used herein, the term "silicone" refers to polymers made up of repeating units of siloxane (-Si-O-Si-), typically with organic side groups such as methyl, phenyl, or vinyl attached to the silicon atoms.

As used herein, the term "elastin" refers to a highly elastic protein composed of repeating units of hydrophobic amino acids such as glycine, valine, alanine, and proline, cross-linked by lysine residues. It forms a fibrous network that allows tissues to stretch and recoil, and it is primarily found in connective tissues such as skin, lungs, and blood vessels.

As used herein, the term "polyurethane" refers to a polymer composed of a chain of organic units joined by urethane linkages, which are formed by the reaction of a diisocyanate with a polyol. The chemical structure can be tailored to produce materials with varying degrees of rigidity and flexibility.

As used herein, the term "mechanical adhesive force" refers to the physical force that adheres two surfaces together through interlocking or frictional contact without the use of chemical bonding. This force arises from the surface roughness and shape of the materials, enhancing their grip or bond through mechanical engagement.

As used herein, the term "cross-linking agent" refers to a chemical compound that facilitates the formation of covalent bonds between polymer chains, thereby linking them together.

As used herein, the term "vulcanizing" refers to a chemical process in which cross-links are formed between polymer chains. This process is often facilitated by heat, pressure and a catalyst and results in the formation of a network structure within the material.

As used herein, the term "grid" refers to a network of intersecting walls that form a series of adjacent spaces or cells. These cells are typically uniform in size and shape, creating a structured and organized layout.

As used herein, the term "wound dressing" refers to a medical material or device applied to a wound to protect it from infection, promote healing, and manage exudate. It is designed to maintain a moist environment, absorb wound fluids, and provide a barrier against external contaminants.

As used herein, the term "shaping" refers to the process of altering the form or configuration of a material to achieve a specific shape or structure, such as forming it into sheets, molds, or other desired configurations. This process involves manipulating the material through techniques like cutting, molding, or pressing to achieve the intended dimensions and properties.

As used herein the term "subject" refers to a human or a non-human mammal. Preferably the subject is human.

### Description of figures

Figure 1 shows the appearance of the composite material. Left side depicts the lower layer in direct contact with wound bed. Right side depicts the upper layer facing away from wound bed).

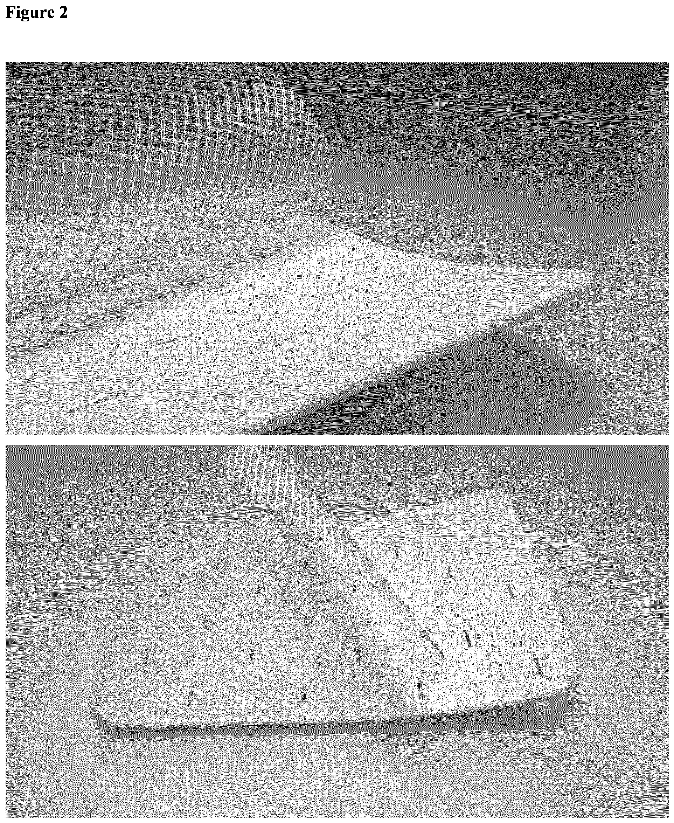

Figure 2 shows the rendered images of the composite material.

### Examples

The invention is now described with reference to the following non-limiting Examples.

### Example 1. Producing the composite material

### 1. Block Formation and Freezing

A suspension of collagen in RO-water with the concentration of 1.8% collagen with respect to the total weight of said composition is mixed with elastin. The collagen/elastin mixture is cast into molds to form blocks. The molds are frozen at specified temperatures (-20°C to -60°C). The frozen blocks are transferred to a lyophilizer to remove moisture via sublimation. The resulting sheets are dry and stable, suitable for storage and further processing.

### 2. Cutting and Fenestration

Lyophilized bricks are cut into approximately A3-sized sheets using a slicing machine. The A3 sheets are placed in a fenestration machine equipped with a stamping die cylinder. This machine creates a pattern of small slits (fenestrations) in the sheets, which helps in wound fluid management and enhances integration with the wound bed.

### 3. Silicone Grid Application

Medical-grade silicone (Silpuran LR 6600/50) is used to create the grid. This silicone is biocompatible and meets ISO 10993 and USP Class VI standards. A CNC machine with a silicone dispenser applies the silicone onto the fenestrated A3 sheets. The machine follows a vector-based CNC program to accurately print the grid structure. This 3D plotting technology builds solid structures layer by layer, offering high precision without the need for specific tooling. Properties such as strand thickness, pore size, and overall grid dimensions are controlled through the software, ensuring a consistent and precise application.

The silicone-coated sheets are placed in a tempering oven temperature 100 °C for an hour to cure the silicone. This heat treatment ensures the silicone fully vulcanizes, providing a lexible, durable matrix that adheres well to collagen. A3 sheets can be optionally cut into various sizes (A4, A6, A8) using a precision cutting machine.

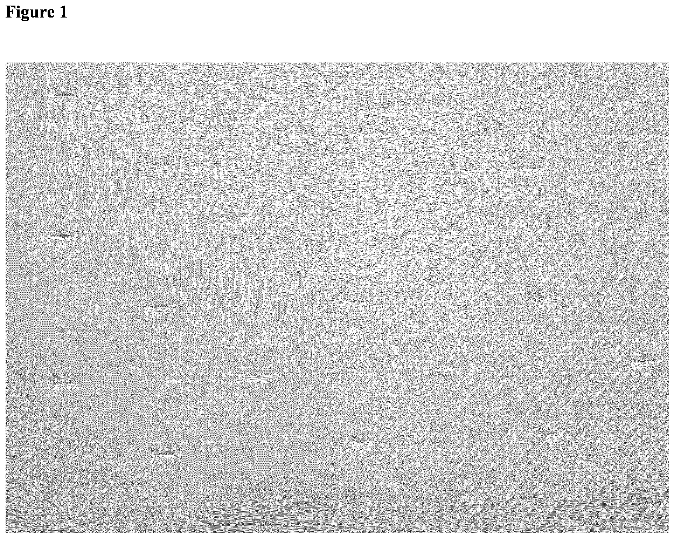

The appearance of the obtained composite material is depicted in Figure 1.

### Example 2. Determination of adhesive force

For determination of adhesive force of silicon layer on collagen sheet material, a peel test is performed with Shimadzu EZ-LX testing machine.

Approximately 40 mm of the silicon layer is carefully removed from the collagen sheet from the short side of the sampling item. The silicon layer is taped to avoid contamination of the clamps.

Determination of adhesion is performed with the force gauge of 50 N. Before starting the machine check for the correct force gauge. Calibration needs to be performed before first measurement.

Clamps are adjusted to 30 mm.

Sample item is put into the clamps with the collagen sheet material clamped into the upper pair and silicon layer is clamped into the lower pair of clamps.

Force is adjusted and the Measurement is started. Maximum adhesive force is determined and recorded by the software automatically after silicon layer is removed from collagen sheet.

### Example 3. Determination of tear strength

The sample moistened with RO water is subjected to pressure at a certain feed rate until it breaks. In this case, the puncture resistance, that is the force that causes the respective sample to break, is determined.

The A3 sheet of a sample is folded to A4 and cut to a width of 5 cm. The thickness of a sample is determined using a thickness gauge. A single specimen is placed on the bottom ring of the the Shimadzu EZ-LX machine and is fixed with the chuck. RO-water is applied to the sample with a spray bottle. Once the sample is completely wetted with RO water, the measurement is started.
Determination of tear strength per sample in cN/mm: R = x_{R} / x_{D}
R: Tear strength per specimen in cN/mm
x_{R}: mean tear strength per sample in cN
x_{D}: average sheet thickness per sample in mm

## Claims

1. A composite material comprising
- a material layer comprising a plurality of cells, wherein each cell is a spatially confined area, and
- a matrix layer,
wherein said material layer comprises an open area in said cells, and wherein said material layer exhibits a proportion of the open area to the entire surface area of the material layer in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm,
wherein said matrix layer comprises a biopolymer selected from the group consisting of collagen, hyaluronic acid, polyacrylate, and polysaccharides, such as alginic acid, nanocellulose, carboxymethyl cellulose or chitosan, or mixtures thereof or salts thereof, and
wherein said material layer and said matrix layer are adhered to one another by means of a mechanical adhesive force of from 0,05 N to 2 N.

2. The composite material according to claim 1, wherein said material layer and said matrix layer are arranged such that the material layer is spaced apart from skin or a wound when applied to the skin.

3. The composite material according to claim 1 or 2, wherein said material layer and said matrix layer are not bound to one another with a cross-linking agent.

4. The composite material according to any of claims 1 to 3, wherein said material layer is **characterized by** the distribution of cells of from 25 to 70 cells, preferably from 40 to 60 cells, more preferably from 45 to 55 cells per 1 cm² of the surface area of the material layer.

5. The composite material according to any of claims 1 to 4, wherein said material layer is **characterized by** the elastic modulus of from 60 kPa to 320 kPa, preferably from 100 kPa to 300 kPa.

6. The composite material according to any of claims 1 to 5, wherein the plurality of cells in said material layer are arranged in a grid.

7. The composite material according to any of claims 1 to 6, wherein said material layer comprises or essentially consists of silicone or polyurethane.

8. The composite material according to claim 7, wherein said material layer comprises or essentially consists of silicone.

9. The composite material according to any of claims 1 to 8, wherein said matrix layer comprises or essentially consists of collagen.

10. The composite material according to claim 9, wherein said matrix layer comprises collagen in an amount of from 85% to 97%, preferably from 90% to 97%, by weight based on the total weight of the matrix layer.

11. The composite material according to any of claims 1 to 10, wherein said matrix layer further comprises elastin.

12. The composite material according to claim 11, wherein said matrix layer comprises elastin in an amount of from 1% to 10%, preferably from 1% to 5%, more preferably 1.5% to 3.0%, in particular, from 1.80 to 2.80 % by weight based on the total weight of the matrix layer.

13. The composite material according to any of claims 1 to 12 for use as a medicament.

14. The composite material according to any of claims 1 to 13 for use in the treatment of acute wounds, such as surgical and traumatic wounds, and chronic wounds, such as ulcers and other chronic wounds.

15. A method for producing a composite material as defined in any of claims 1 to 13, comprising the following steps:
a) Providing a suspension of a biopolymer in an aqueous solution, wherein the concentration of the biopolymer in said suspension is in the range of from 1.0 to 4.0%, preferably from 1.40% to 2.00% more preferably from 1.55 to 1.80% with respect to the total weight of said suspension, wherein the biopolymer is selected from the group consisting of collagen, hyaluronic acid, polyacrylate, and polysaccharides, such as alginic acid, nanocellulose, carboxymethyl cellulose or chitosan, or mixtures thereof or salts thereof;
b) Shaping said suspension to provide a matrix layer;
c) Providing a material which is **characterized by** the elastic modulus of from 60 kPa to 320 kPa, preferably from 100 kPa to 300 kPa;
d) Applying said material onto said matrix layer using a computer numerical control (CNC) machine to provide a composite material, wherein said CNC machine is configured to provide a material layer in said composite material with a proportion of open area to the entire surface area in the range of from 10% to 50%, preferably from 20% to 40%, and a thickness in the range of from 0.10 to 2 mm, preferably from 0.30 to 0.70 mm, more preferably from 0.40 to 0.60 mm, even more preferably from 0.45 to 0.55 mm; and
e) Vulcanizing the material obtained in step d), in particular by exposing said material to an elevated temperature and/or UV Irradiation.
